# FASCICULE DE BREVET EUROPEEN

(11) **EP 1 078 611 B1**
(45) Date de publication et mention de la délivrance du brevet: **09.06.2004**
(21) Numéro de dépôt: 00400710.0
(22) Date de dépôt: 15.03.2000
(51) Int. Cl.: A61F 2/06

(54) **Système de mise en place d'une prothèse intravasculaire**
Anbringungssystem für eine intravaskuläre Prothese
System for deployment of an intravascular prosthesis

(30) Priorité: 24.08.1999 FR 9910733
(43) Date de publication de la demande: 28.02.2001
(73) Titulaire: Novatech SA, 06130 Grasse le Plan (FR)
(72) Inventeur: Fedida, José, 06370 Mouans-Sartoux (FR)
(74) Mandataire: Hauer, Bernard

(56) Documents cités:
- EP-A- 0 418 677
- EP-A- 0 536 610
- DE-U- 29 717 110
- US-A- 5 534 007

## Description

La présente invention concerne un dispositif pour provoquer le largage dans un conduit humain ou animal d'un objet, en particulier une prothèse, ainsi qu'un système d'implantation comportant un tel dispositif et un cathéter porteur de l'objet.

Le document DE-U-297 17 110 est consideré comme l'état de la technique le plus proche.

Bien que non exclusivement, la présente invention s'applique plus particulièrement à l'implantation d'une prothèse dans une artère du corps humain, et notamment dans l'aorte abdominale.

On sait que l'implantation d'une prothèse artérielle, telle que celles divulguées par exemple par les documents EP-0 461 791, WO-95/21592 et EP-0 880 948, est généralement réalisée à l'aide d'un cathéter qui comporte notamment, de façon connue :
- une gaine porteuse qui comprend, en partie distale, des moyens de dilatation, par exemple un nez dilatateur avec ou sans ballon gonflable, et qui porte ladite prothèse ;
- une gaine intermédiaire poussoir qui entoure partiellement ladite gaine porteuse et qui est susceptible de coulisser par rapport à cette dernière ; et
- une gaine externe de protection qui recouvre, au moins partiellement, l'extrémité distale de ladite gaine porteuse, qui entoure partiellement ladite gaine intermédiaire et qui est susceptible de coulisser par rapport à cette dernière.

De façon connue, l'implantation de la prothèse dans une artère présente généralement les trois phases successives suivantes, qui peuvent notamment être suivies par radiographie, en prévoyant par exemple des éléments radio-opaques sur le cathéter et/ou la prothèse :
- une phase initiale, pendant laquelle on amène la prothèse au moins à proximité de son futur emplacement dans l'artère, à l'aide du cathéter, dont les trois gaines précitées sont rendues solidaires les unes des autres. A titre d'illustration, pour implanter une prothèse aortique bifurquée, par exemple du type de celle décrite dans le document EP-0 461 791, dans l'aorte abdominale entre les artères iliaques et les artères rénales, on fait généralement pénétrer le cathéter dans le corps humain au niveau des artères fémorales et on amène la partie distale dudit cathéter, qui porte la prothèse, dans l'aorte, via les artères iliaques ;
- une phase de largage, pendant laquelle on libère la prothèse dans l'artère. A cet effet, il faut notamment faire coulisser la gaine externe de protection par rapport à la gaine intermédiaire de telle sorte que ladite gaine externe libère la partie distale du cathéter et notamment la zone où se trouve la prothèse ; et
- une phase de retrait du cathéter, hors du corps humain.

Dans le cadre de la présente invention, on s'intéresse plus particulièrement, bien que non exclusivement, à ladite phase de largage.

Généralement, pour la mise en oeuvre de cette phase de largage, le chirurgien qui réalise l'implantation de la prothèse ou l'un de ses assistants, fait coulisser manuellement la gaine externe par rapport à la gaine intermédiaire, en prenant en main deux parties extracorporelles desdites gaines, de manière à obtenir la libération précitée de la prothèse.

Comme il est réalisé manuellement et de ce fait généralement par à-coups, ce coulissement (et donc également le largage de la prothèse) est peu précis et irrégulier. De plus, il est difficile, et parfois même impossible, à mettre à oeuvre car :
- les gaines sont généralement glissantes ; et surtout
- il est nécessaire de produire une force élevée, notamment pour larguer de grandes prothèses, telles que les prothèses aortiques bifurquées précitées.

La présente invention a pour objet de remédier à ces inconvénients. Elle concerne un dispositif permettant de réaliser facilement dans un conduit humain ou animal un largage qui est précis et sans à-coups et ne présente aucun risque de blesser ledit conduit, d'un objet et notamment d'une prothèse, ledit objet étant porté et amené dans ledit conduit humain ou animal par un cathéter qui comprend une première et une seconde gaines (gaine intermédiaire et gaine externe de protection, dans l'exemple précité) partiellement introduites dans ledit conduit, montées de façon télescopique et susceptibles de coulisser l'une par rapport à l'autre et qui réalise le largage dudit objet en réponse à un coulissement relatif approprié entre lesdites première et seconde gaines.

A cet effet, selon l'invention, ledit dispositif est remarquable en ce qu'il comporte :
- un boîtier ;
- des premiers moyens de fixation susceptibles de fixer une partie extracorporelle de ladite première gaine sur ledit boîtier ;
- un chariot mobile monté sur ledit boîtier de manière à pouvoir être déplacé par rapport à ce dernier ;
- des seconds moyens de fixation susceptibles de fixer une partie extracorporelle de ladite seconde gaine sur ledit chariot mobile ; et
- des moyens d'entraînement commandables susceptibles de déplacer ledit chariot mobile, par rapport audit boîtier.

Ainsi, grâce à l'invention, le coulissement relatif entre lesdites première et seconde gaines est réalisé par le dispositif conforme à l'invention. Plus précisément, ce coulissement est engendré par le déplacement du chariot mobile (auquel est fixée une gaine) par rapport au boîtier (auquel est fixée l'autre gaine). Ceci est réalisé automatiquement ou par la génération d'une force réduite (selon le mode de réalisation considéré des moyens d'entraînement, comme précisé ci-dessous) sans commune mesure avec la force précitée devant être exercée habituellement par un chirurgien sans l'utilisation du dispositif conforme à l'invention.

Par conséquent, comme on le verra plus en détail ci-dessous, le largage de l'objet peut être réalisé :
- de façon continue et régulière, c'est-à-dire sans à-coups ;
- sans risquer de blesser le conduit ; et
- de façon très précise,
et ceci par la seule et simple maîtrise du déplacement dudit chariot mobile.

Dans le cadre de la présente invention, les moyens d'entraînement utilisés à cet effet peuvent être réalisés de différentes manières.

En particulier, de façon avantageuse :
- dans un premier mode de réalisation, lesdits moyens d'entraînement comportent une vis sans fin qui coopère avec un taraudage prévu dans le chariot et qui est mue par un moteur commandable ;
- dans un second mode de réalisation, lesdits moyens d'entraînement comportent un système à crémaillère qui agit sur le chariot et qui est également mû par un moteur commandable ;
- dans un troisième mode de réalisation, lesdits moyens d'entraînement comportent :
   . un moyen élastique, de préférence un ressort, qui est monté de façon élastiquement contraint entre le boîtier et le chariot de manière à pouvoir déplacer ce dernier par rapport audit boîtier ; et
   . un arrêt commandable susceptible de bloquer le déplacement dudit chariot mobile par rapport audit boîtier ; et
- dans un quatrième mode de réalisation, lesdits moyens d'entraînement comportent un système à levier susceptible d'être actionné ou commandé manuellement.

De plus, dans lesdits premier et second modes de réalisation, ledit moteur, de préférence un moteur électrique alimenté par pile ou par le secteur, peut être commandé automatiquement par un élément programmable, par exemple une puce électronique. Dans ce cas, la phase de largage peut être réalisée de façon automatique, selon un programme préétabli.

En outre, avantageusement, ledit chariot est monté de façon mobile sur des rails de guidage, de préférence rectilignes, qui sont solidaires dudit boîtier.

Par ailleurs, selon l'invention, de façon avantageuse, le boîtier dudit dispositif :
- comporte une fenêtre transparente permettant de voir de l'extérieur une partie (se trouvant à l'intérieur dudit boîtier) d'au moins l'une desdites gaines, ce qui permet de suivre visuellement le coulissement relatif entre les deux gaines, et donc la progression de la phase de largage dudit objet ; et/ou
- est réalisé en deux parties au moins partiellement séparables et susceptibles d'être rendues solidaires l'une de l'autre, ce qui permet de monter facilement un cathéter dans ledit boîtier et donc dans le dispositif conforme à l'invention ; et/ou
- présente une forme allongée comprenant une zone de prise manuelle adaptée à la main d'un utilisateur dudit dispositif, ce qui permet notamment de faciliter la prise en main et la manipulation dudit dispositif.

La présente invention concerne également un système pour implanter un objet, en particulier une prothèse, dans un conduit humain ou animal, notamment dans une artère.

Selon l'invention, ledit système est remarquable en ce qu'il comporte :
- un cathéter qui :
   . peut porter et amener ledit objet dans ledit conduit humain ou animal ;
   . comprend une première et une seconde gaines susceptibles d'être partiellement introduites dans ledit conduit, montées de façon télescopique et susceptibles de coulisser l'une par rapport à l'autre ; et
   . peut réaliser le largage dudit objet en réponse à un coulissement relatif approprié entre lesdites première et seconde gaines ; et
- un dispositif tel que celui précité, pour provoquer le largage dudit objet dans ledit conduit humain ou animal, en engendrant ledit coulissement relatif entre les première et seconde gaines.

En outre, de façon avantageuse, au moins l'une desdites gaines comporte une valve anti-reflux, ce qui permet d'empêcher dans le cas d'une implantation dans une artère ou un vaisseau sanguin, un écoulement sanguin hors de ladite artère ou vaisseau sanguin, via ledit cathéter.

De plus, avantageusement, ladite valve anti-reflux est susceptible de coopérer avec des moyens de fixation du dispositif conforme à l'invention, pour simplifier et améliorer la fixation des première et seconde gaines audit dispositif.

Par ailleurs, pour faciliter le suivi visuel de la phase de largage, par exemple à travers une fenêtre transparente du boîtier, tel que précité, au moins l'une desdites gaines, de préférence la gaine intermédiaire, comporte de façon avantageuse au moins une graduation de type usuel sur sa face externe.

Les figures du dessin annexé feront bien comprendre comment l'invention peut être réalisée. Sur ces figures, des références identiques désignent des éléments semblables.

Les figures 1 à 3 montrent un premier mode de réalisation d'un dispositif conforme à l'invention, respectivement en vue de face, de dessus (le boîtier étant à chaque fois ouvert) et en perspective.

Les figures 4 et 5 illustrent schématiquement un cathéter respectivement en position juste avant le largage de l'objet et en position larguée.

Les figures 6 et 7 illustrent schématiquement, respectivement en vue de face et en vue de dessus (le boîtier étant à chaque fois ouvert), un deuxième mode de réalisation d'un dispositif conforme à l'invention.

Les figures 8 et 9 illustrent schématiquement un dispositif conforme à l'invention, respectivement dans un troisième et un quatrième modes de réalisation.

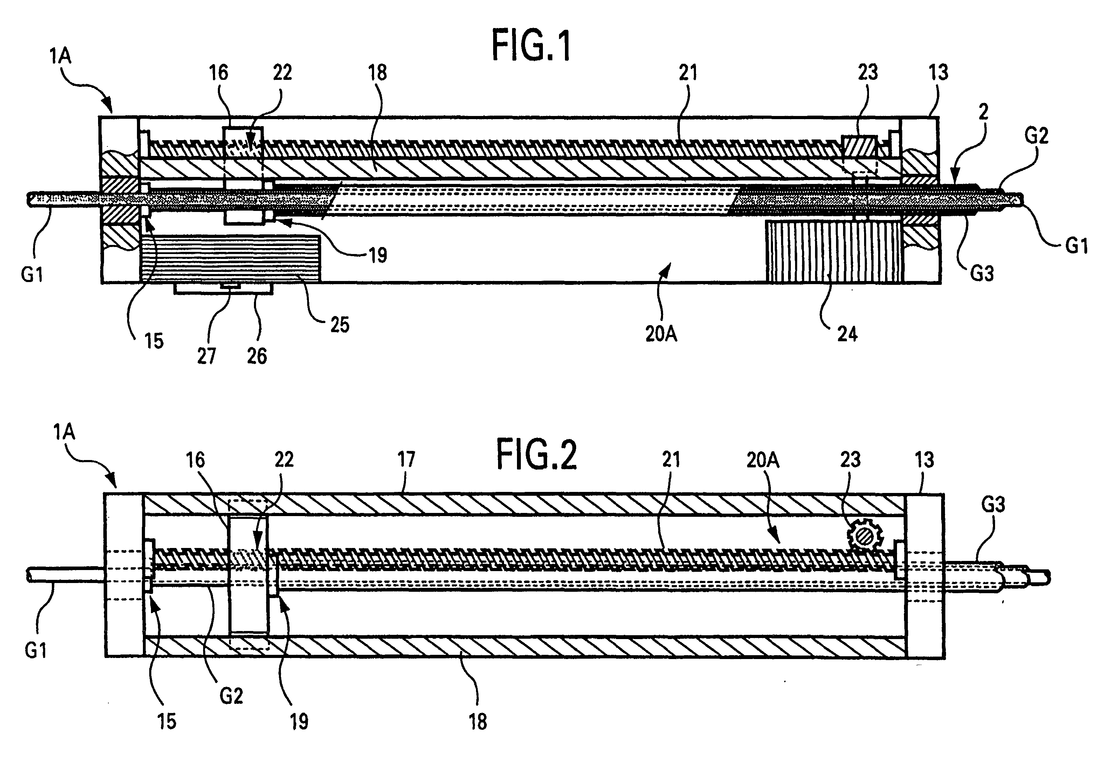

Le dispositif 1A, 1B, 1C, 1D conforme à l'invention et représenté schématiquement, selon un premier mode de réalisation 1A, sur les figures 1 à 3, est destiné à provoquer le largage dans un conduit humain ou animal d'un objet, en particulier une prothèse.

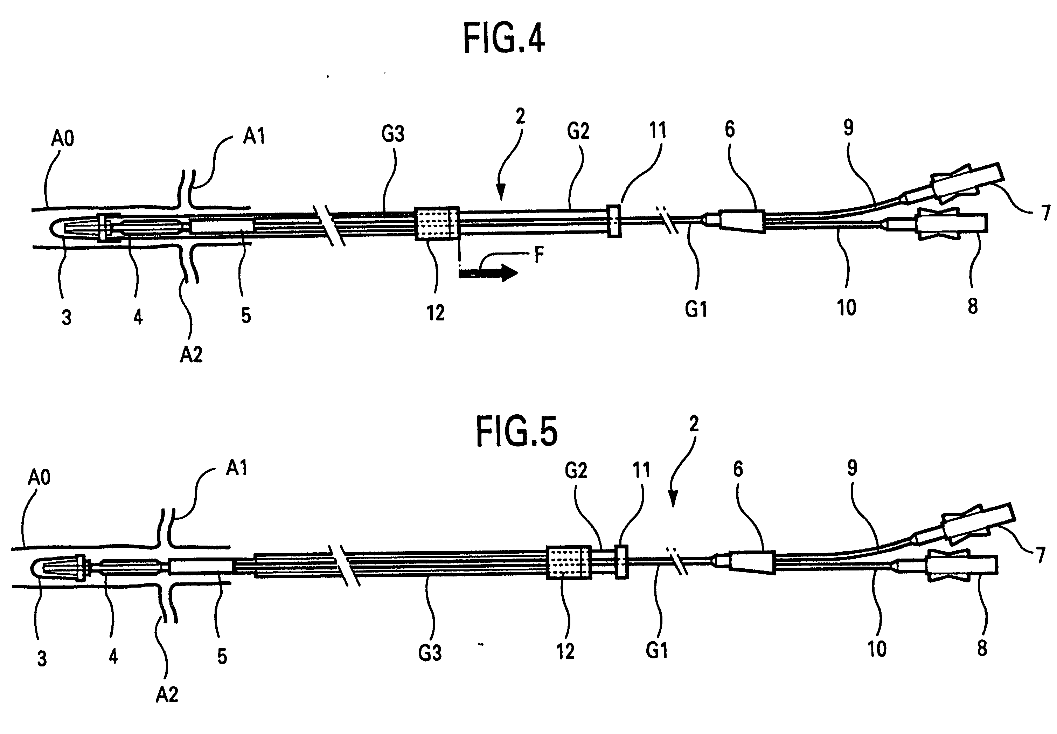

A cet effet, ledit dispositif 1A, 1B, 1C, 1D est associé à un cathéter 2 représenté sur les figures 4 et 5 pour former un système d'implantation conforme à l'invention.

De façon connue, un tel cathéter 2 ou introducteur comporte une gaine porteuse G1 qui est réalisée sous forme d'un tube à double lumière qui comprend, en partie distale, successivement un nez dilatateur 3, un ballon de dilatation 4 et une prothèse 5 destinée à être implantée dans une artère AO du corps humain, par exemple dans l'aorte abdominale, notamment au niveau de l'embranchement d'autres artères A1 et A2, par exemple des artères rénales.

Ladite gaine porteuse G1 comprend de plus, en partie proximale, un moyen de dérivation 6 auquel sont reliés deux moyens de connection 7 et 8 de type connu "luer lock" de manière à créer deux voies 9 et 10, l'une pour le gonflage du ballon de dilatation 4 et l'autre pour la rigidification de la gaine porteuse G1, par exemple au moyen d'une tige métallique non représentée et introduite dans ladite gaine porteuse G1.

Ledit cathéter connu 2 comporte de plus :
- une gaine intermédiaire G2 entourant la gaine G1 dans la partie distale jusqu'au niveau de la prothèse 5 et susceptible de coulisser sur ladite gaine G1 ; et
- une gaine externe G3 de protection entourant la gaine G2 dans la partie distale jusqu'au nez dilatateur 3 et susceptible de coulisser sur ladite gaine G2.

Les gaines G1 à G3 sont donc montées de façon télescopique.

Lors de l'implantation de la prothèse 5, on amène, de façon connue, la partie distale du cathéter 2 dans l'aorte AO de sorte que la prothèse 5 se trouve au moins à proximité de son emplacement futur, comme illustré sur la figure 4. A partir de cette position, il convient de réaliser ensuite : d'abord le largage de la prothèse 5, puis le retrait du cathéter 2 hors du corps humain.

De façon connue, le largage est réalisé par le retrait ou le coulissement selon la flèche F représentée sur la figure 4 de la gaine G3 par rapport à la gaine G2, ce qui permet de libérer la partie distale du cathéter 2, comprenant notamment le ballon 4 et la prothèse 5, comme illustré sur la figure 5.

Chacune des gaines G2 et G3 comporte de plus à son extrémité proximale une collerette 11, 12 précisée ci-dessous et permettant notamment de fixer la gaine correspondante G2, G3 respectivement sur la gaine G1, G2 qui lui est interne.

Le dispositif 1A, 1B, 1C, 1D est destiné plus particulièrement à engendrer le retrait ou coulissement de la gaine G3 par rapport à la gaine G2, à partir de la position illustrée sur la figure 4, pour provoquer la libération et le largage de la prothèse 5, comme représenté sur la figure 5.

Plus généralement, ledit dispositif 1A, 1B, 1C, 1D est destiné à engendrer le coulissement relatif entre deux gaines d'un cathéter en vue de libérer, dans un conduit humain ou animal, un objet quelconque porté par ledit cathéter.

A cet effet, en vue de créer ledit coulissement relatif, il est envisageable, dans une application particulière non représentée de l'invention, de déplacer, non pas la gaine G3 externe, mais la gaine G2, en la poussant dans ladite gaine G3.

Selon l'invention, ledit dispositif 1A à 1D comporte, comme représenté par exemple sur les figures 1 à 3 dans le premier mode de réalisation 1A :
- un boîtier 13, 14 susceptible d'être traversé, au moins partiellement, par le cathéter 2 ;
- des moyens de fixation 15 précisés ci-dessous pour fixer sur ledit boîtier 13, 14 une partie qui est extracorporelle (dans les positions des figures 4 et 5) de la gaine G2, de préférence la collerette 11 de cette gaine G2, comme précisé ci-dessous ;
- un chariot mobile 16 monté par l'intermédiaire de rails de guidage 17 et 18 sur ledit boîtier 13, 14 ;
- des moyens de fixation 19 précisés ci-dessous, pour fixer une partie qui est extracorporelle (dans les positions des figures 4 et 5) de la gaine G3, de préférence la collerette 12 comme indiqué ci-dessous, sur ledit chariot mobile 16 ; et
- des moyens d'entraînement commandables 20A, 20B, 20C, 20D susceptibles de déplacer ledit chariot mobile 16, par rapport audit boîtier 13, 14.

Par conséquent, lorsqu'il veut réaliser le largage de la prothèse 5 à partir de la position du cathéter 2 représentée sur la figure 4, ledit cathéter 2 étant lié au dispositif 1A à 1D conforme à l'invention de la manière précitée, le chirurgien qui réalise l'implantation de la prothèse 5 ou l'un de ses assistants doit simplement commander les moyens d'entraînement 20A, 208, 20C et 20D. Ces derniers engendrent alors le déplacement du chariot mobile 16 par rapport au boîtier 13, 14 et ainsi le coulissement entre lesdites gaines G3 et G2 qui sont fixées respectivement sur le chariot mobile 16 et sur le boîtier 13, 14.

Le largage est donc réalisé automatiquement ou tout au plus par la génération d'une force réduite (selon le mode de réalisation considéré des moyens d'entraînement 20A à 20D, comme précisé ci-dessous) sans commune mesure avec la force qui aurait dû être exercée par le chirurgien sans l'utilisation du dispositif conforme à l'invention.

Par conséquent, grâce à l'invention, le largage est réalisé :
- de façon régulière et continu, sans à-coups, ce qui permet de mieux maîtriser la phase de largage et d'éviter tout risque de blessure de la paroi de l'artère AO ;
- de façon très précise, la phase de largage pouvant notamment être suivie visuellement et/ou par radiographie et même être préprogrammée, comme précisé ci-dessous ; et
- sans aucun effort physique ou, tout au plus, avec un effort physique très réduit du chirurgien, même lorsque la prothèse 5 à larguer est très grande.

Dans le cadre de la présente invention, les moyens d'entraînement peuvent être réalisés de différentes manières.

Dans le premier mode de réalisation 1A représenté sur les figures 1 à 3, lesdits moyens d'entraînement 20A comportent une vis sans fin 21 qui est montée sur ledit boîtier 13, qui coopère avec un taraudage 22 prévu dans le chariot 16 et qui est mue par une roue dentée 23 entraînée par un moteur 24, en particulier un moteur électrique.

Dans le cas d'un moteur électrique 24, ce dernier peut être alimenté en courant électrique, soit par l'intermédiaire du secteur (non représenté), soit par l'intermédiaire d'au moins une pile électrique 25. Dans ce cas, le boîtier 13 comporte de plus :
- un interrupteur 26 accessible de l'extérieur, pour commander ledit moteur 24 ; et
- un voyant de charge 27 susceptible de s'allumer ou de clignoter, lorsque le niveau de charge de la pile électrique 25 passe au-dessous d'un niveau prédéterminé.

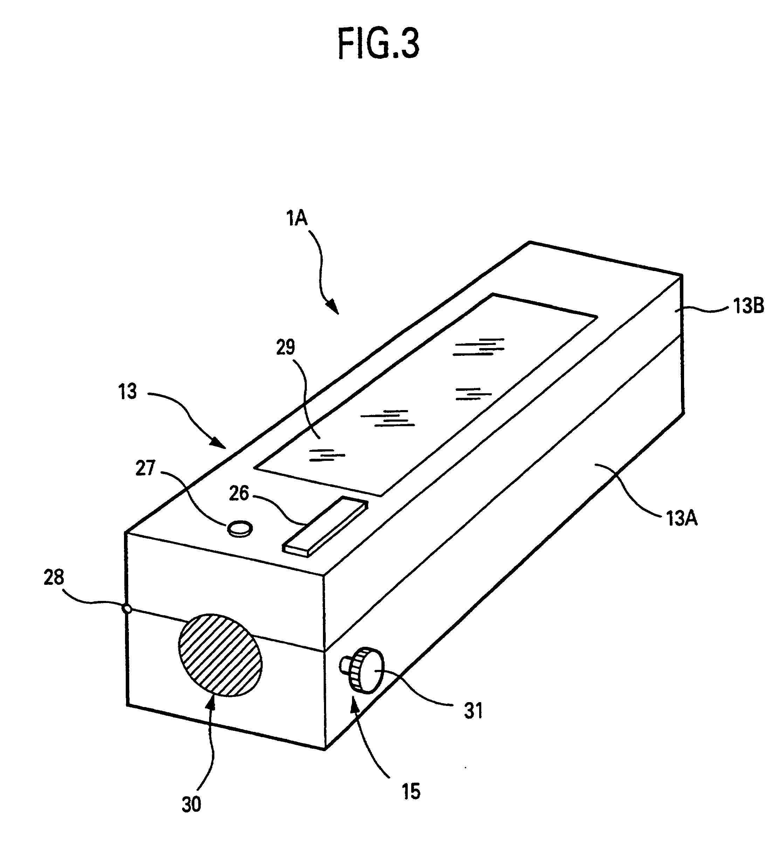

Comme on peut le voir sur la figure 3, le boîtier 13 est réalisé de préférence en deux parties 13A et 13B liées ensemble par des charnières 28, ce qui permet de monter facilement un cathéter 2 dans ledit boîtier 13 en faisant simplement pivoter la partie 13B par rapport à la partie 13A pour ouvrir ledit boîtier 13.

Le boîtier 13 comporte de plus :
- une fenêtre transparente 29 permettant de suivre visuellement le coulissement relatif entre les gaines G2 et G3 et donc la phase de largage. A cet effet, au moins l'une desdites gaines G2, G3, de préférence la gaine G2, comporte sur sa face externe au moins une graduation usuelle ; et
- des ouvertures 30 prévues longitudinalement de part et d'autre du boîtier 13, pour permettre le passage du cathéter 2, les diamètres desdites deux ouvertures 30 étant adaptés de préférence à ceux respectivement des gaines G1 et G3.

Dans un mode de réalisation particulier, les moyens de fixation 15 comportent une vis 31. Dans le cadre de la présente invention, ils peuvent également comporter un clip ou tout autre élément de fixation connu et non représenté. Il en est de même des moyens de fixation 19.

De préférence, lesdits moyens de fixation 15 et 19 coopèrent respectivement avec les collerettes 11 et 12, qui sont formées en correspondance, pour réaliser la fixation des gaines G2 et G3.

Lesdites collerettes 11 et 12 comportent, de plus, des valves anti-reflux de manière à empêcher tout écoulement sanguin hors de l'artère AO, via le cathéter 2.

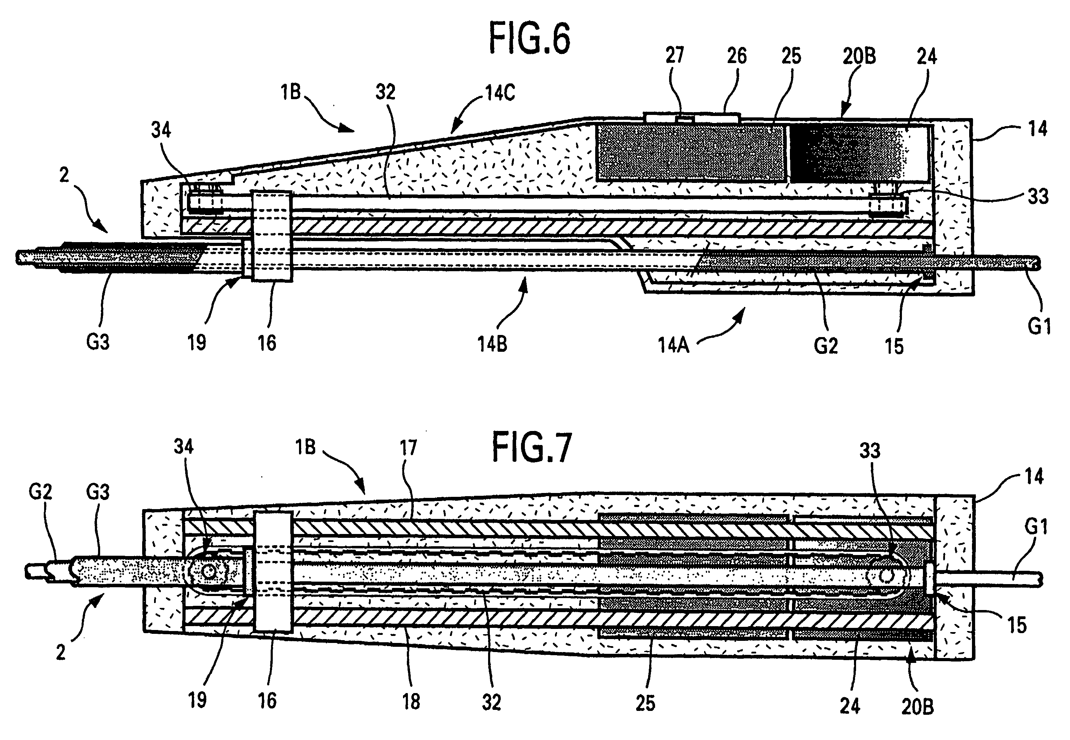

Par ailleurs, dans le second mode de réalisation 1B représenté sur les figures 6 et 7, les moyens d'entraînement 20B comportent un système à crémaillère qui comprend une crémaillère ou courroie crantée 32 agissant sur le chariot mobile 16 formé de façon appropriée, entraînée en rotation par une roue dentée 33 mue par le moteur 24 et agencée entre ladite roue dentée 33 mue et une roue dentée 34 libre en rotation.

En outre, le boîtier 14 comporte à l'une des extrémités une zone de prise 14A adaptée à la main d'un utilisateur du dispositif 1B, ce qui facilite la manipulation de ce dernier. A l'autre extrémité dudit boîtier 14, l'une 14C des faces 14B, 14C est biseautée et l'autre face 14B est formée de sorte que le chariot 16 se trouve partiellement à l'extérieur dudit boîtier 14, ainsi que partiellement les parties de gaines G2 et G3 situées entre les collerettes 11 et 12, ce qui facilite le suivi visuel du coulissement relatif entre lesdites gaines G2 et G3.

On notera en outre que, dans les modes de réalisation 1A et 1B, le moteur 24 peut être commandé automatiquement par un élément programmable non représenté, par exemple une puce électronique intégrée. Ainsi, le largage de la prothèse 5 peut être mis en oeuvre automatiquement selon un programme préétabli.

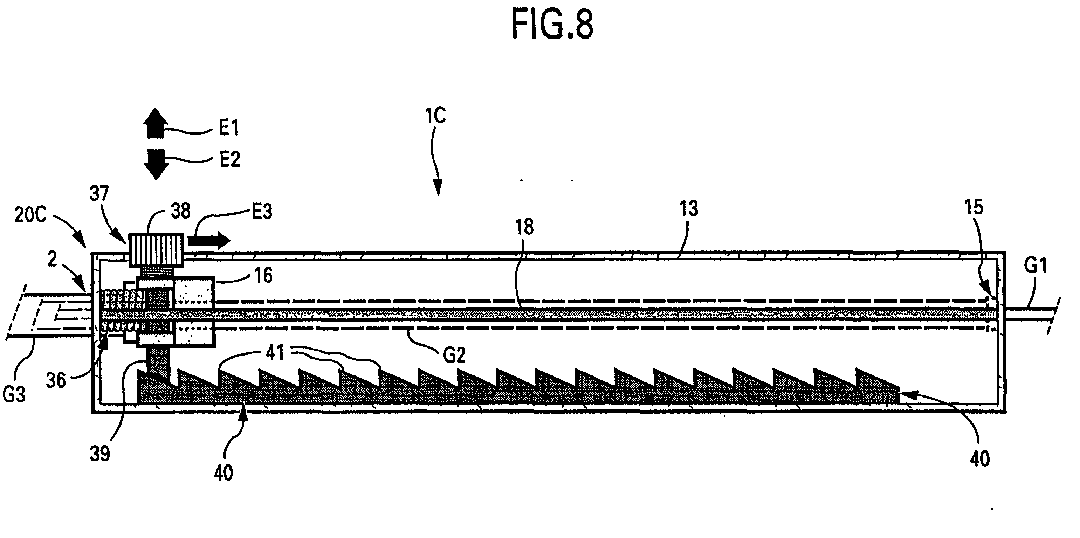

Par ailleurs, dans le troisième mode de réalisation 1C représenté sur la figure 8, les moyens d'entraînement 20C comportent :
- un moyen élastique 36, de préférence un ressort, qui est monté de façon élastiquement contraint entre le boîtier 13 et le chariot mobile 16 et qui est susceptible de faire coulisser ledit chariot mobile 16 sur les rails de guidage 18 ; et
- un arrêt 37 commandable, qui est susceptible de bloquer le déplacement dudit chariot mobile 16 par rapport audit boîtier 13.

Dans un mode de réalisation particulier tel que représenté, ledit arrêt 37 comporte un bouton 38 susceptible d'être tiré ou poussé, comme illustré respectivement par des flèches E1 et E2. Ce bouton 38 est solidaire d'une languette rigide 39 susceptible d'agir dans une barrette 40 dentée (ou crantée), qui est fixée au fond du boîtier 13. L'extrémité libre de ladite languette 39 est biseautée de manière à s'adapter à la forme des dents 41 de ladite barrette 40.

Lorsque, à partir de la position représentée sur la figure 8, on tire le bouton 38 dans le sens de la flèche E1, la languette 39 est libérée de la barrette 40 de sorte que l'arrêt du chariot 16 est débloqué. Ce dernier est alors contraint à se déplacer dans le sens de la flèche E3 sous l'action du moyen élastique 36, et ceci jusqu'à ce qu'il arrive à l'extrémité opposée ou jusqu'à ce que le bouton 38 soit de nouveau enfoncé dans le sens de la flèche E2.

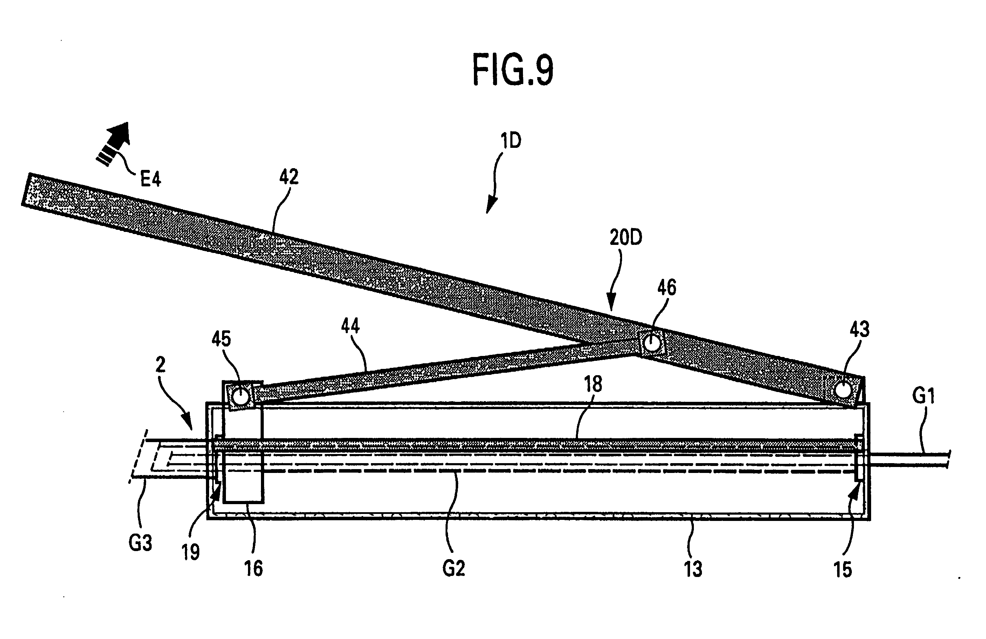

En outre, dans le quatrième et dernier mode de réalisation 1D représenté sur la figure 9, les moyens d'entraînement 20D comportent un système à levier qui comprend :
- un levier 42 actionnable, comme illustré par une flèche E4, et monté de façon pivotante sur le boîtier 13 par l'intermédiaire d'un pivot 43 ; et
- un bras intermédiaire 44 monté de façon articulée sur le chariot 16 et le levier 42, respectivement par l'intermédiaire d'articulations 45 et 46.

Par conséquent, comme cela ressort clairement de la figure 9, un déplacement ou actionnement, de préférence manuel, de l'extrémité libre du levier 42 engendre le déplacement du chariot 16, par l'intermédiaire du bras 44. On notera que, même lorsque l'actionnement précité est réalisé manuellement, la force à exercer sur le levier 42 reste très faible, en raison notamment de l'effet de levier obtenu grâce à l'invention.

## Revendications

1. Dispositif pour provoquer le largage dans un conduit humain (AO) ou animal d'un objet (5), en particulier une prothèse, ledit objet (5) étant porté et amené dans ledit conduit humain (AO) ou animal par un cathéter (2) qui comprend une première et une seconde gaines (G2, G3) partiellement introduites dans ledit conduit (AO), montées de façon télescopique et susceptibles de coulisser l'une par rapport à l'autre et qui peut réaliser le largage dudit objet en réponse à un coulissement relatif approprié entre lesdites première et seconde gaines (G2, G3), ledit dispositif comportant
- un boîtier (13, 14) ;
- un chariot mobile (16) monté sur ledit boîtier (13, 14) de manière à pouvoir être déplacé par rapport à ce dernier ;
- des seconds moyens de fixation (19) susceptibles de fixer une partie extracorporelle de ladite seconde gaine (G3) sur ledit chariot mobile (16) ; et
- des moyens d'entraînement commandables (20A, 20B, 20C, 20D) susceptibles de déplacer ledit chariot mobile (16), par rapport audit boîtier (13, 14),
**caractérisé en ce qu'**il comporte
- des premiers moyens de fixation (15) susceptibles de fixer une partie extracorporelle de ladite première gaine (G2) sur ledit boîtier (13, 14).

2. Dispositif selon la revendication 1,
**caractérisé en ce que** lesdits moyens d'entraînement (20A) comportent une vis sans fin (21) qui coopère avec un taraudage (22) prévu dans ledit chariot (16) et qui est mue par un moteur (24) commandable.

3. Dispositif selon la revendication 1,
**caractérisé en ce que** lesdits moyens d'entraînement (20B) comportent un système à crémaillère (32, 33, 34) qui agit sur ledit chariot (16) et qui est mû par un moteur (24) commandable.

4. Dispositif selon l'une des revendications 2 et 3,
**caractérisé en ce que** ledit moteur (24) est commandé automatiquement par un élément programmable.

5. Dispositif selon la revendication 1,
**caractérisé en ce que** lesdits moyens d'entraînement (20C) comportent :
- un moyen élastique (36) qui est monté de façon élastiquement contraint entre ledit boîtier (13) et ledit chariot (16) ; et
- un arrêt (37) commandable susceptible de bloquer le déplacement dudit chariot mobile (16) par rapport audit boîtier (13).

6. Dispositif selon la revendication 1,
**caractérisé en ce que** lesdits moyens d'entraînement (20D) comportent un système à levier (42, 43, 44, 45, 46) susceptible d'être actionné manuellement.

7. Dispositif selon l'une quelconque des revendications 1 à 6,
**caractérisé en ce que** ledit chariot (16) est monté de façon mobile sur des rails de guidage (17, 18) solidaires dudit boîtier (13, 14).

8. Dispositif selon l'une quelconque des revendications précédentes,
**caractérisé en ce que** ledit boîtier (13) comporte une fenêtre transparente (29) permettant de voir de l'extérieur une partie d'au moins l'une desdites gaines (G2, G3), située à l'intérieur dudit boîtier (13).

9. Dispositif selon l'une quelconque des revendications précédentes,
**caractérisé en ce que** ledit boîtier (13) est réalisé en deux parties (13A, 13B) au moins partiellement séparables et susceptibles d'être rendues solidaires l'une de l'autre.

10. Dispositif selon l'une quelconque des revendications précédentes,
**caractérisé en ce que** ledit boîtier (14) présente une forme allongée comprenant une zone de prise manuelle (14A) adaptée à la main d'un utilisateur dudit dispositif (1B).

11. Système pour implanter un objet, en particulier une prothèse (5), dans un conduit humain (AO) ou animal,
**caractérisé en ce qu'**il comporte :
- un cathéter (2) qui :
. peut porter et amener ledit objet (5) dans ledit conduit humain (AO) ou animal ;
. comprend une première et une seconde gaines (G2, G3) susceptibles d'être partiellement introduites dans ledit conduit (AO), montées de façon télescopique et susceptibles de coulisser l'une par rapport à l'autre ; et
. peux réaliser le largage dudit objet (5) en réponse à un coulissement relatif approprié entre lesdites première et seconde gaines (G2, G3) ; et
- un dispositif (1A, 1B, 1C, 1D) tel que celui spécifié sous l'une quelconque des revendications 1 à 10, pour provoquer le largage dudit objet (5) dans ledit conduit humain (AO) ou animal, en engendrant ledit coulissement relatif entre lesdites première et seconde gaines (G2, G3).

12. Système selon la revendication 11,
**caractérisé en ce qu'**au moins l'une desdites gaines (G2, G3) comporte une valve anti-reflux (11, 12).

13. Système selon la revendication 12,
**caractérisé en ce que** ladite valve anti-reflux (11, 12) est susceptible de coopérer avec des moyens de fixation (15, 19) dudit dispositif (1A, 1B, 1C, 1D) destiné à provoquer le largage dudit objet (5).

14. Système selon l'une quelconque des revendications 11 à 13,
**caractérisé en ce qu'**au moins l'une desdites gaines (G2, G3) comporte au moins une graduation sur sa face externe.

## Patentansprüche

1. Vorrichtung zur Freisetzung eines Gegenstandes (5), insbesondere einer Prothese in einem menschlichen oder tierischen Kanal (AO), wobei der Gegenstand (5) von einem Katheter (2) getragen wird, und in den menschlichen oder tierischen Kanal (AO) eingeführt wird, wobei der Katheter (2) eine erste und eine zweite Hülle (G2, G3) umfasst, die teilweise in den Kanal (AO) eingeführt werden, auf teleskopische weise montiert sind und in Bezug zueinander gleiten können, und als Reaktion auf ein entsprechendes relatives Gleiten zwischen der ersten und der zweiten Hülle (G2, G3) die Freisetzung des Gegenstandes durchführen kann, wobei die Vorrichtung umfasst:
- ein Gehäuse (13, 14);
- eine bewegliche Laufvorrichtung (16), die an dem Gehäuse (13, 14) montiert ist, derart, dass sie in Bezug auf das Letztgenannte bewegt werden kann;
- zweite Befestigungsmittel (19), die einen extrakorporalen Teil der zweiten Hülle (G3) an der beweglichen Laufvorrichtung (16) befestigen können; und
- steuerbare Antriebsmittel (20A, 20B, 20C, 20D), die die bewegliche Laufvorrichtung (16) in Bezug auf das Gehäuse (13, 14) bewegen können,
**dadurch gekennzeichnet, dass** sie erste Befestigungsmittel (15) umfasst, die einen extrakorporalen Teil der ersten Hülle (G2) an dem Gehäuse (13, 14) befestigen können.

2. Vorrichtung nach Anspruch 1, **dadurch gekennzeichnet, dass** die Antriebsmittel (20A) eine Schraube ohne Ende (21) umfassen, die mit einem in der Laufvorrichtung (16) vorgesehen Innengewinde (22) zusammenarbeitet und die durch einen steuerbaren Motor (24) angetrieben wird.

3. Vorrichtung nach Anspruch 1, **dadurch gekennzeichnet, dass** die Antriebsmittel (20B) ein zahnriemensystem (32, 33, 34) umfassen, das auf die Laufvorrichtung (16) einwirkt und durch einen steuerbaren Motor (24) angetrieben wird.

4. Vorrichtung nach einem der Ansprüche 2 und 3, **dadurch gekennzeichnet, dass** der Motor (24) automatisch durch ein programmierbares Element gesteuert wird.

5. Vorrichtung nach Anspruch 1, **dadurch gekennzeichnet, dass** die Antriebsmittel (20C) umfassen:
- ein elastisches Mittel (36), das auf unter Federkraft stehende Weise zwischen dem Gehäuse (13) und der Laufvorrichtung (16) montiert ist; und
- eine steuerbare Stoppvorrichtung (37), die die Bewegung der beweglichen Laufvorrichtung (16) in Bezug auf das Gehäuse (13) stoppen kann.

6. Vorrichtung nach Anspruch 1, **dadurch gekennzeichnet, dass** die Antriebsmittel (20D) ein Hebelsystem (42, 43, 44, 45, 46) umfassen, das manuell betätigt werden kann.

7. Vorrichtung nach einem der Ansprüche 1 bis 6, **dadurch gekennzeichnet, dass** die Laufvorrichtung (16) auf bewegliche Weise auf Führungsschienen (17, 18) montiert ist, die mit dem Gehäuse (13, 14) fest verbunden sind.

8. Vorrichtung nach einem der vorhergehenden Ansprüche, **dadurch gekennzeichnet, dass** das Gehäuse (13) ein durchsichtiges Fenster (29) umfasst, das es ermöglicht, einen Teil von mindestens einer der Hüllen (G2, G3), der sich im Inneren des Gehäuses (13) befindet, von außen zu sehen.

9. Vorrichtung nach einem der vorhergehenden Ansprüche, **dadurch gekennzeichnet, dass** das Gehäuse (13) in zwei Teilen (13A, 13B) ausgeführt ist, die mindestens teilweise voneinander getrennt und fest miteinander verbunden werden können.

10. Vorrichtung nach einem der vorhergehenden Ansprüche, **dadurch gekennzeichnet, dass** das Gehäuse (14) eine längliche Form aufweist, die einen Bereich zum manuellen Ergreifen (14A) umfasst, der an die Hand eines Benutzers der Vorrichtung (1B) angepasst ist.

11. System zur Implantation eines Gegenstandes, insbesondere einer Prothese (5), in einem menschlichen oder tierischen Kanal (AO), **dadurch gekennzeichnet, dass** es umfasst:
- einen Katheter (2), der
. den Gegenstand (5) tragen und in den menschlichen oder tierischen Kanal (AO) einführen kann; eine erste und eine zweite Hülle (G2, G3) umfasst, die teilweise in den Kanal (AO) eingeführt werden können, auf teleskopische Weise montiert sind und in Bezug zueinander gleiten können; und
. als Reaktion auf ein entsprechendes relatives Gleiten zwischen der ersten und der zweiten Hülle (G2, G3) die Freisetzung des Gegenstandes (5) durchführen kann; und
- eine Vorrichtung (1A, 1B, 1C, 1D), wie die in einem der Ansprüche 1 bis 10 spezifizierte, zur Freisetzung des Gegenstandes (5) in dem menschlichen oder tierischen Kanal (AO), indem sie das relative Gleiten zwischen der ersten und der zweiten Hülle (G2, G3) hervorruft.

12. System nach Anspruch 11, **dadurch gekennzeichnet, dass** mindestens eine der Hüllen (G2, G3) ein Antirefluxventil (11, 12) umfasst.

13. System nach Anspruch 12, **dadurch gekennzeichnet, dass** das Antirefluxventil (11, 12) mit Befestigungsmitteln (15, 19) der Vorrichtung (1A, 1B, 1C, 1D) zusammenarbeiten kann, die dazu bestimmt ist, die Freisetzung des Gegenstandes (5) hervorzurufen.

14. System nach einem der Ansprüche 11 bis 13, **dadurch gekennzeichnet, dass** mindestens eine der Hüllen (G2, G3) an ihrer Außenseite mindestens eine Skala umfasst.

## Claims

1. Device for causing the release of an object (5), particularly a prosthesis, into a human passage (AO) or animal passage, the said object (5) being carried and delivered into the said human passage (AO) or animal passage by a catheter (2) which comprises a first sheath (G2) and a second sheath (G3) which are partially introduced into the said passage (AO) and mounted telescopically and able to slide one with respect to the other, which catheter can release the said object in response to an appropriate relative sliding of the said first and second sheaths (G2, G3), the said device comprising:
- a casing (13, 14);
- a mobile carriage (16) mounted on the said casing (13, 14) so that it can be moved with respect thereto;
- second fixing means (19) capable of fixing an extracorporal part of the said second sheath (G3) to the said mobile carriage (16); and
- controllable drive means (20A, 20B, 20C, 20D) capable of moving the said mobile carriage (16) with respect to the said casing (13, 14), **characterized in that** it comprises
- first fixing means (15) capable of fixing an extracorporal part of the said first sheath (G2) to the said casing (13, 14).

2. Device according to Claim 1, **characterized in that** the. said drive means (20A) comprise an endless screw (21) which collaborates with a tapping (22) made in the said carriage (16) and which is moved by a controllable motor (24).

3. Device according to Claim 1, **characterized in that** the said drive means (20B) comprise a rack system (32, 33, 34) which acts on the said carriage (16) and which is moved by a controllable motor (24).

4. Device according to one of Claims 2 and 3, **characterized in that** the said motor (24) is controlled automatically by a programmable element.

5. Device according to Claim 1, **characterized in that** the said drive means (20C) comprise:
- an elastic means (36) which is mounted so that it is elastically constrained between the said casing (13) and the said carriage (16); and
- a controllable stop (37) capable of preventing the movement of the said mobile carriage (16) with respect to the said casing (13).

6. Device according to Claim 1, **characterized in that** the said drive means (20D) comprise a lever system (42, 43, 44, 45, 46) which can be actuated manually.

7. Device according to any one of Claims 1 to 6, **characterized in that** the said carriage (16) is mounted so that it can move on guide rails (17, 18) secured to the said casing (13, 14).

8. Device according to any one of the preceding claims, **characterized in that** the said casing (13) has a transparent window (29) which allows part of at least one of the said sheaths (G2, G3), which part is located inside the said casing (13), to be seen from the outside.

9. Device according to any one of the preceding claims, **characterized in that** the said casing (13) is made in two parts (13A, 13B) which are at least partially separable and which can be secured together.

10. Device according to any one of the precding claims, **characterized in that** the said casing (14) has an elongate shape comprising a handgrip region (14A) tailored to the hand of someone using the said device (1B).

11. System for implanting an object, particularly a prosthesis (5), in a human passage (AO) or animal passage, **characterized in that** it comprises:
- a catheter (2) which:
· can carry and deliver the said object (5) into the said human passage (AO) or animal passage;
· comprises a first sheath (G2) and a second sheath (G3) which can be partially introduced into the said passage (AO) and are mounted telescopically and able to slide one with respect to.the other; and
· can release the said object (5) in response to an appropriate relative sliding of the said first and second sheaths (G2, G3); and
- a device (1A, 1B, 1C, 1D) as specified in any one of Claims 1 to 10, for causing the release of the said object (5) in the said human passage (AO) or animal passage by causing the said relative sliding of the said first and second sheaths (G2, G3).

12. System according to Claim 11, **characterized in that** at least one of the said sheaths (G2, G3) comprises an anti-reflux valve (11, 12).

13. System according to Claim 12, **characterized in that** the said anti-reflux valve (11, 12) is capable of collaborating with fixing means (15, 19) of the said device (1A, 1B, 1C, 1D) intended for causing the release of the said object (5).

14. System according to any one of Claims 11 to 13, **characterized in that** at least one of the said sheaths (G2, G3) has at least one graduation on its external face.
